# EUROPEAN PATENT SPECIFICATION

(11) **EP 4 623 885 B1**
(45) Date of publication and mention of the grant of the patent: **25.11.2026**
(21) Application number: 24186127.7
(22) Date of filing: 02.07.2024
(51) Int. Cl.: A61H 7/00, A61M 21/00

(54) **SOUNDPROOF AUTOMATED CABIN SPA WITH MASSAGE CHAIR**
SCHALLDICHTES AUTOMATISIERTES KABINENSPA MIT MASSAGESTUHL
SPA DE CABINE AUTOMATISÉE INSONORISÉE DOTÉ D'UNE CHAISE DE MASSAGE

(30) Priority: 28.03.2024 IT 202400001402 U
(43) Date of publication of application: 01.10.2025
(73) Proprietor: Tylki, Frederick Jan, Tunbridge Wells, Kent TN1 2FD (GB)
(72) Inventor: Tylki, Frederick Jan, Tunbridge Wells, Kent TN1 2FD (GB)
(74) Representative: LS-MP von Puttkamer Berngruber Loth Spuhler

(56) References cited:
- AU-A1- 2021 432 514
- CN-A- 107 049 671
- CN-A- 112 147 932
- CN-A- 113 062 632
- CN-A- 113 143 750
- CN-B- 114 469 094
- US-A1- 2018 320 394

## Description

### Technical field

This invention pertains to the field of wellness and relaxation technology, specifically focusing on the development of a unique, soundproof automated cabin spa equipped with a massage chair, designed for on-the-go relaxation in public and private settings.

### Background of the invention

The main existing technologies in the field of relaxation and wellness within busy environments are known to be the following:
- automated massage chairs, that give the physical relaxation that a user might need, but lack privacy and soundproofing, not enabling them to fully disconnect from the busy environment they could be placed in;
- massage centers and SPAs, which can guarantee privacy, relaxation, soundproofing, and disconnection. Those centers, however, have to deal with high management costs such as rent, salaries, materials, etc. This does not only imply complex management but, of course, also leads to a higher price for the end user, becoming hardly cost-effective;
- soundproof cabins, which are intended to isolate the user from external sounds, e.g., during calls or meetings. These cabins help users to isolate themselves from external noise, but do not grant any service made specifically to relax them physically and mentally. Exemplary cabins are known from AU2021432514A1, US2018/320394A1, CN107049671A and CN112147932A.

An object of the present invention is to address the need for a private, tranquil space where individuals can momentarily escape their hectic routines and reconnect with themselves.

This object is reached, according to the invention, by means of a portable, self-contained unit that provides a comprehensive relaxation experience, integrating soundproofing, automated access, and immersive relaxation technologies. The integration of a soundproof cabin, automated features, and a massage chair into a single, cohesive unit represents a novel approach, enhancing user experience by offering privacy, relaxation, and convenience in busy environments such as shopping centers, airports, and corporate spaces, as well as private homes.

Upon entering the soundproof cabin, users are insulated from external noise, enabling them to seat on a massage chair that offers a full body massage, including reflexology. The ambiance is further enhanced with nature videos and sounds via an internal monitor, speakers, and a 360-degree projector. Privacy is assured through tinted windows and a bamboo fence that delineates the cabin's perimeter.

Users can access the cabin through an automated payment system, selecting the experience duration of 10 or 20 minutes. Additional features include external advertising and informative monitors, motion detectors that activate ventilation and voice commands, and safety measures such as a CCTV camera and an emergency exit mechanism.

The invention is a synthesis of several components: an automated payment kiosk, information and advertising TV monitors, a bamboo privacy fence, an automated door system, a soundproof cabin, a latest generation massage chair, a security camera, internal speakers, a 360-degree projector, an automated spa ambiance air freshener, and PIR motion detectors. The combination of these elements, governed by an integrated hardware and software system, creates a unique product that enhances user experience by offering a secluded, immersive relaxation space in otherwise busy or stressful environments.

### Detailed Description of the Invention

Further features and advantages of the present invention will become apparent from the following detailed description, given merely by way of non-limiting example with reference to the appended drawings, wherein:

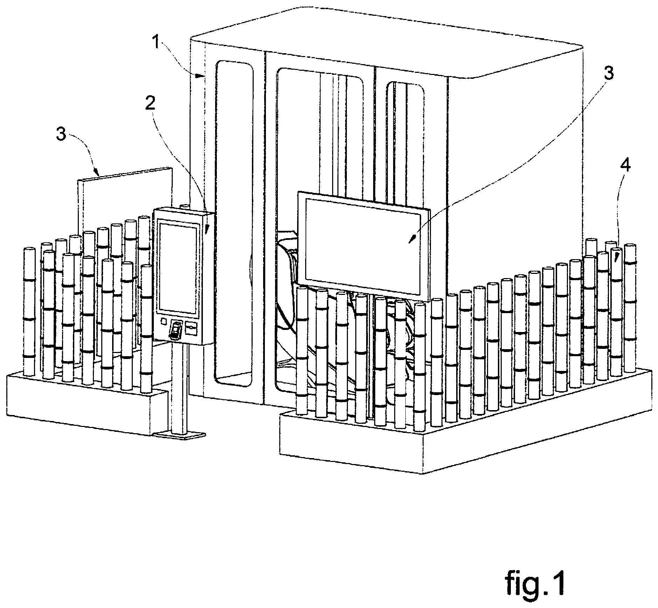

- Figure 1 is a schematic perspective view of a soundproof automated cabin spa, according to an embodiment of the present invention;

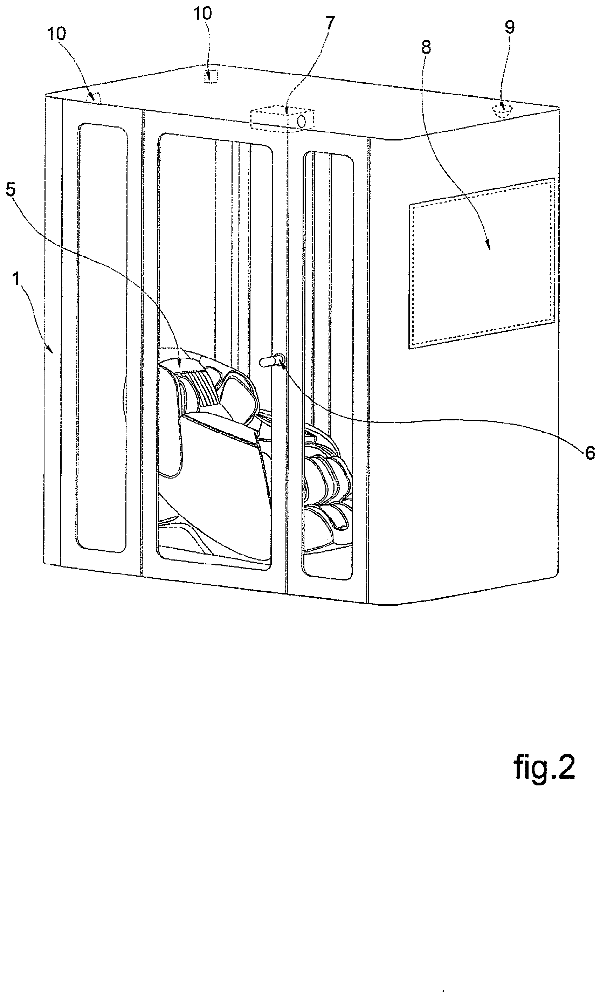

- Figure 2 is a schematic perspective view of a soundproof automated cabin, according to an embodiment of the present invention;

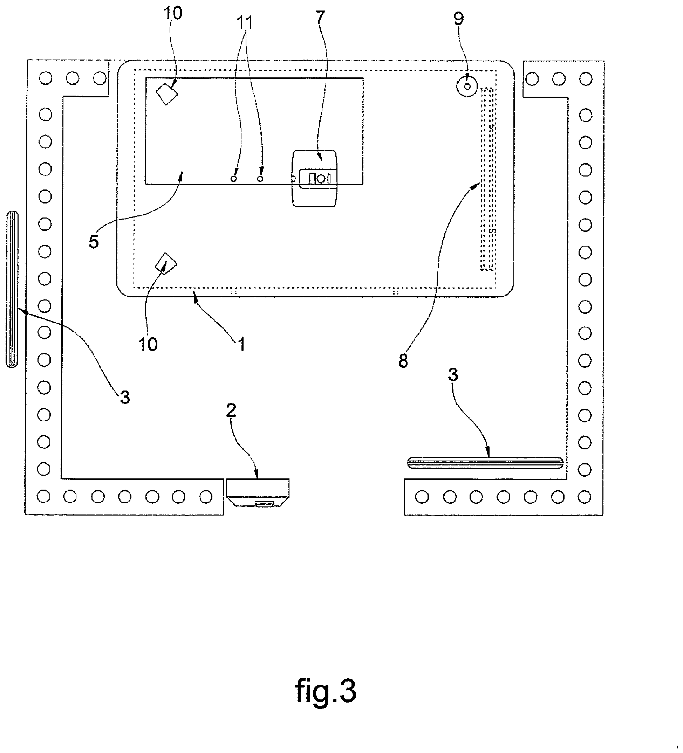

- Figure 3 is a schematic sectional view from the top of the soundproof automated cabin spa of Figure 1, according to an embodiment of the present invention; and

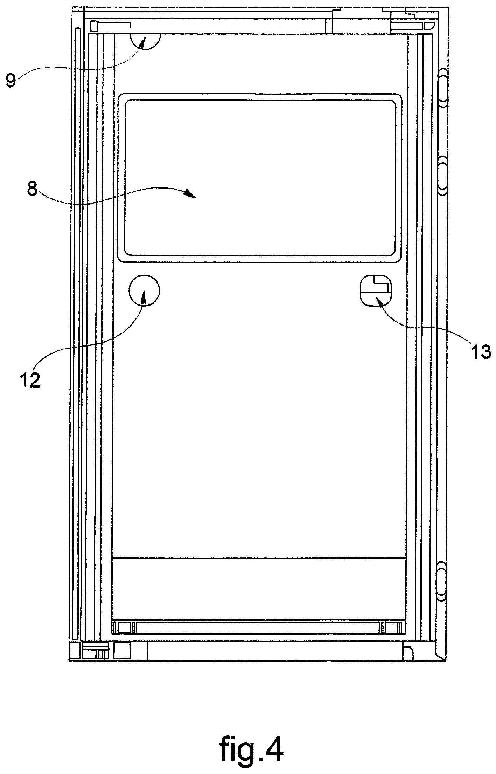

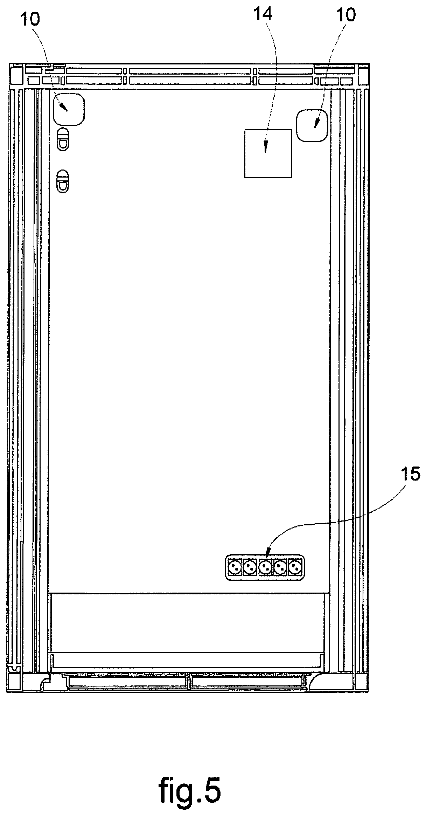

- Figures 4 and 5 are schematic side views of a soundproof automated cabin, according to an embodiment of the present invention.

Referring by way of example to the figures, an automated and soundproof cabin spa provided with a massage chair is a cabin (1) made of sound-absorbing materials suitable to ensure acoustic insulation from the outside, so as to create a private and detached environment for theuser. The aforementioned cabin (1) has a ventilation adjustment system and an air circulation system and having power sockets 15 at the ends of the walls of the cabin (1) characterized in that the cabin (1) contains:
- a full-body massage chair (5) with an automated and customizable adjustment system;
- a CCTV surveillance and security camera (9);
- a 360-degree internal projector (7);
- two passive infrared motion detectors (11) adapted to enable the automatic activation of voice commands and the ventilation system;
- speakers (10) positioned at the top of the cabin (1) and connected to the passive infrared motion detectors (11);
- an automatic fragrance diffuser;
- a television 8; and - an emergency button that allows communication with a phone operator in case of emergency.

The cabin (1) is preferably made by sound-absorbing materials, metal covers, felt, plywood, isolating materials, and soundproof glass. Said cabin (1) is equipped with a massage chair (5), and may have adjustable interior lighting, a CCTV camera (9), a spa ambiance air freshener (14), an emergency button (12), two motion detectors (PIR) (11), a 43" TV (8), a 360° projector (7), speakers (10) for voice commands, and essential visual information such as an emergency and customer service contact number.

The massage chair (2) is preferably made of plastic, metal, massage rollers, airbags, and an electronic interface, in such a way as to offer customizable massages through a user-friendly control device and is conveniently upholstered in durable, comfortable synthetic leather.

The security camera (9) comprises plastic case, lenses, and electronics, and is preferably remotely connected for user safety.

The internal cabin speakers (10) preferably deliver relaxing sounds and information messages (such as welcome messages, how-to info, time left, and time-limit-reached notification).

The internal 360-Degree projector (7) is preferably located on the ceiling, and is intended to create a relaxing ambiance by projecting soothing images onto the cabin walls.

The automated spa ambient air freshener (14) is configured to deliver a gentle, natural fragrance, improving the relaxing experience.

The PIR (passive infrared) motion detectors (1)1 are configured for activating the cabin's ventilation system and voice commands, upon a user entering the cabin (1).

According to an embodiment, the automated and soundproof spa cabin with massage chair comprises two passive infrared motion detectors (11) configured for directly activating the speakers (10), the projector (7), and the fragrance diffuser.

According to an embodiment, the automated and soundproof cabin spa with a massage chair comprises a door with an automated opening from the outside, directly connected to a payment system, the door being configured to be opened from the inside of the cabin (1) to allow a user to get out of the cabin at any time.

The door can be operated by means of an automatic door opening system (6), integrated with the payment system. This safety-compliant locking mechanism is configured in such a way as to ensure that users can enter after purchasing their session and exit freely at any time with an antipanic system.

The payment system can be operated by means of a payment system kiosk (2), which combines a touch monitor, an electronic payment device, and a banknote insertion slot, facilitating cabin access and massage chair activation. Upon payment for a chosen duration (e.g., 10 or 20 minutes), it is configured to send an MDB protocol signal to unlock the cabin door and triggers voice instructions for the user inside. The 15" touchscreen displays a tutorial video and payment options, indicating cabin occupancy and remaining time for the next user.

According to an embodiment, the automated and soundproof cabin spa with a massage chair comprises a bamboo fence (4), surrounding the perimeter of the cabin (1) to ensure the user's privacy during the service offered by the cabin, one or more advertising monitors (3), aimed at promoting the product, and a payment station, for activating the automated door opening and the product experience.

The bamboo fence (4) is preferably 1-meter high and encircles the cabin (1), offering privacy while maintaining visibility of the cabin to encourage use by others.

The TV monitors for information and advertising (3) may comprise two exterior 43" monitors for displaying promotional content in a loop. Inside, another 43" monitor 8 enhances the relaxing experience with high-definition (4K) nature images and sounds. The external monitor will be preferably located 106 cm (the bottom part of the screen) from the ground, at eye view from the passersby, and above the screen will be preferably featured a commercial logo. The internal screen is preferably located at eye view from the chair user (when the chair (5) is reclined), so starting from a height of 133 cm (bottom part of the screen) above the ground. For what concerns the hardware and software components of the automated elements included in the cabin spa with a massage chair, the latter may be provided with a system which integrates display touchscreens, CPUs, Wi-Fi routers, I/O boards (Interface Input-output board), banknote readers, POS systems, door sensors, and automated locks through a network of cables and software, automating the cabin spa experience.

In particular, the hardware may include a touchscreen display at the payment kiosk (2), a CPU with LAN for internet connection and system communication, a Wi-Fi router for network access, an I/O board for interfacing with various components, a CCTalk protocol banknote reader, a certified POS system for electronic payments, motion and door sensors for user interaction, 1VIDB signal cables (allows to send 1VIDB protocol messages between the systems), and a panic button for emergencies.

The software used can be Win11, VBnet, C, C++, Assembler, Http, Json, SQL.

This invention provides a novel solution to improve personal wellness by offering a moment of peace and relax amidst the hustle and bustle of daily life.

### List of references

Soundproof automated cabin 1
Payment system kiosk 2
Advertising & Information screen 3
Bamboo fence 4
Massage chair 5
Automatic door opening system 6
360° Projector 7
Internal screen 8
Security camera 9
Internal cabin speakers 10
PIR (Passive Infrared) motion detector 11
Emergency button 12
Light dimming and ventilation speed controller 13
Automated spa ambient air freshener 14
Electrical outlets 15

## Claims

1. Automated and soundproof spa cabin with massage chair, wherein it comprises a cabin (1) made of sound-absorbing materials suitable to ensure acoustic insulation from the outside, so as to create a private and detached environment for the individual, said cabin (1) having a ventilation adjustment system and an air circulation system and having power sockets (15) at the ends of the walls thereof, **characterized in that** the cabin (1) contains:
- a full-body massage chair (5) with an automated and customizable adjustment system;
- a CCTV surveillance and security camera (9);
- a 360-degree internal projector (7);
- two passive infrared motion detectors (11) adapted to enable the automatic activation of voice commands and the ventilation system;
- speakers (10) positioned at the top of the cabin (1) and connected to the passive infrared motion detection (11);
- an automatic fragrance diffuser;
- a television 8; and
- an emergency button that allows communication with a phone operator in case of emergency.

2. Automated and soundproof spa cabin with massage chair according to claim 1, **characterized in that** it comprises a door with an automated opening from the outside directly connected to a payment system, the door being openable from the inside to allow a user to get out of the cabin (1) at any time.

3. Automated and soundproof spa cabin with massage chair according to claim 1, further comprising a bamboo fence (4), surrounding the perimeter of the cabin (1) to ensure the user's privacy during the service offered by the cabin, one or more advertising monitors (3), aimed at promoting the product, and a payment station, configured for activating the automated door opening system and the product experience.

4. Automated and soundproof spa cabin with massage chair according to claims 1 and 2 , comprising an automated passive infrared sensor system consisting of two passive infrared motion detectors (11) on the ceiling of the cabin (1).

5. Automated and soundproof spa cabin with massage chair according to claims 1 and 4, comprising two passive infrared motion detectors (11) configured for directly activating the speakers (10), the projector (7), and the fragrance diffuser.

## Patentansprüche

1. Automatisierte und schalldichte Spa-Kabine mit Massagesessel, wobei sie eine Kabine (1) aus schallabsorbierenden Materialien umfasst, die geeignet sind, eine akustische Isolierung gegenüber der Außenumgebung sicherzustellen, um so eine private und abgeschiedene Umgebung für die Person zu schaffen, wobei die Kabine (1) ein Lüftungsregelungssystem und ein Luftumwälzsystem sowie an den Enden ihrer Wände Steckdosen (15) aufweist, **dadurch gekennzeichnet, dass** die Kabine (1) Folgendes enthält:
- einen Ganzkörper-Massagesessel (5) mit einem automatisierten und individuell anpassbaren Verstellsystem;
- eine CCTV-Überwachungs- und Sicherheitskamera (9);
- einen 360-Grad-Innenprojektor (7);
- zwei passive Infrarot-Bewegungsmelder (11), die dazu ausgelegt sind, die automatische Aktivierung von Sprachbefehlen und des Lüftungssystems zu ermöglichen;
- Lautsprecher (10), die im oberen Bereich der Kabine (1) angeordnet und mit der passiven Infrarot-Bewegungserfassung (11) verbunden sind;
- einen automatischen Duftstoffdiffusor;
- einen Fernseher 8; und
- einen Notfallknopf, der im Notfall die Kommunikation mit einem Telefonisten ermöglicht.

2. Automatisierte und schalldichte Spa-Kabine mit Massagesessel nach Anspruch 1, **dadurch gekennzeichnet, dass** sie eine Tür mit einer automatisierten Öffnung von außen umfasst, die direkt mit einem Bezahlsystem verbunden ist, wobei die Tür von innen geöffnet werden kann, um es einem Benutzer zu ermöglichen, die Kabine (1) jederzeit zu verlassen.

3. Automatisierte und schalldichte Spa-Kabine mit Massagesessel nach Anspruch 1, ferner umfassend einen Bambuszaun (4), der den Umfang der Kabine (1) umgibt, um die Privatsphäre des Benutzers während der von der Kabine angebotenen Dienstleistung sicherzustellen, einen oder mehrere Werbemonitore (3), die zur Bewerbung des Produkts bestimmt sind, und eine Bezahlstation, die zum Aktivieren des automatisierten Türöffnungssystems und des Produkterlebnisses konfiguriert ist.

4. Automatisierte und schalldichte Spa-Kabine mit Massagesessel nach den Ansprüchen 1 und 2, umfassend ein automatisiertes passives Infrarot-Sensorsystem, bestehend aus zwei passiven Infrarot-Bewegungsmeldern (11) an der Decke der Kabine (1).

5. Automatisierte und schalldichte Spa-Kabine mit Massagesessel nach den Ansprüchen 1 und 4, umfassend zwei passive Infrarot-Bewegungsmelder (11), die zum direkten Aktivieren der Lautsprecher (10), des Projektors (7) und des Duftdiffusors konfiguriert sind.

## Revendications

1. Cabine de spa automatisée et insonorisée équipée d'un fauteuil de massage, comprenant une cabine (1) réalisée en matériaux insonorisants permettant d'assurer l'isolation acoustique vis-à-vis de l'extérieur, afin de créer un environnement privé et isolé pour l'individu. La cabine (1) est équipée d'un système de régulation de la ventilation et d'un système de circulation d'air, ainsi que de prises électriques (15) situées aux extrémités de ses parois, **caractérisée en ce que** la cabine (1) contient :
- un fauteuil de massage intégral (5) doté d'un système de réglage automatique et personnalisable ;
- une caméra de vidéosurveillance et de sécurité (9) ;
- un projecteur interne à 360 degrés (7) ;
- deux détecteurs de mouvement infrarouge passifs (11) conçus pour permettre l'activation automatique des commandes vocales et du système de ventilation ;
- des haut-parleurs (10) placés dans la partie supérieure de la cabine (1) et reliés au détecteur de mouvement infrarouge passif (11) ;
- un diffuseur de parfum automatique ;
- un téléviseur 8 ; et
- un bouton d'urgence permettant de contacter un opérateur téléphonique en cas d'urgence.

2. Cabine de spa automatisée et insonorisée équipée d'un fauteuil de massage selon la revendication 1, **caractérisée en ce qu'**elle comprend une porte à ouverture automatisée depuis l'extérieur, directement reliée à un système de paiement, cette porte pouvant être ouverte de l'intérieur pour permettre à un utilisateur de sortir de la cabine (1) à tout moment.

3. Cabine de spa automatisée et insonorisée avec fauteuil de massage selon la revendication 1, comprenant en outre une clôture en bambou (4), entourant le périmètre de la cabine (1) afin de garantir l'intimité de l'utilisateur pendant le service proposé par la cabine, un ou plusieurs écrans publicitaires (3), destinés à promouvoir le produit, et une borne de paiement, configurée pour activer le système d'ouverture automatisée de la porte et l'expérience produit.

4. Cabine de spa automatisée et insonorisée, équipée d'un fauteuil de massage selon les revendications 1 et 2, comprenant un système automatisé de capteurs infrarouges passifs composé de deux détecteurs de mouvement infrarouges passifs (11) fixés au plafond de la cabine (1).

5. Cabine de spa automatisée et insonorisée, équipée d'un fauteuil de massage selon les revendications 1 et 4, comprenant deux détecteurs de mouvement infrarouge passifs (11) configurés pour activer directement les haut-parleurs (10), le projecteur (7) et le diffuseur de parfum.
